# EUROPÄISCHE PATENTANMELDUNG

(11) **EP 3 501 446 A1**
(43) Veröffentlichungstag der Anmeldung: **26.06.2019**
(21) Anmeldenummer: 17209843.6
(22) Anmeldetag: 21.12.2017
(51) Int. Cl.: A61C 7/00, A61B 90/00, A61B 34/20

(54) **VERFAHREN ZUM VERGLEICHENDEN DARSTELLEN EINES IST- UND EINES SOLL-ZUSTANDES**

(71) Anmelder: GC International AG, 6003 Luzem (CH)
(72) Erfinder: JESENKO, Juergen, 9587 Riegersdorf (AT); VON SEE, Constantin, 3500 Krems/Imbach (AT)
(74) Vertreter: Beer & Partner Patentanwälte KG

(57) **Zusammenfassung**

Bei einem Verfahren zum Darstellen eines Ist-Zustandes von intraoralen Strukturen und eines Soll-Zustandes, wobei der Soll-Zustand einen Verlauf einer geplanten Bohrung innerhalb einer intraoralen Struktur enthält, wobei die geplante Bohrung optisch erfassbare und optisch nicht erfassbare Bereiche beinhaltet, beinhaltet der Ist-Zustand eine Referenzbohrung. Das Referenzobjekt ist in der Referenzbohrung positioniert und eine Geometrie eines Referenzobjektes und eine Geometrie des Soll-Zustandes werden zur Verfügung gestellt. Dann werden eine Oberflächengeometrie der intraoralen Strukturen und zeitgleich die Oberflächengeometrie des Referenzobjektes wenigstens bereichsweise erfasst. Daraus wird das räumliche Verhältnis der intraoralen Strukturen zum Referenzobjekt ermittelt. Aus dem räumlichen Verhältnis wird der Verlauf der Referenzbohrung ermittelt. Der Verlauf der Referenzbohrung und der Verlauf der geplanten Bohrung werden schließlich in einer Darstellung dargestellt.

## Beschreibung

Die Erfindung betrifft ein Verfahren zum Darstellen eines Ist-Zustandes von intraoralen Strukturen und eines Soll-Zustandes, wobei der Soll-Zustand einen Verlauf einer geplanten Bohrung innerhalb einer intraoralen Struktur enthält und wobei die geplante Bohrung optisch erfassbare und optisch nicht erfassbare Bereiche beinhaltet.

In der Implantologie, insbesondere der oralen Implantologie, ist es aus dem Stand der Technik bekannt, verschiedene Bildgebungsverfahren zu kombinieren, um zu einer umfassenden Darstellung der Situation zu gelangen und einen bestmöglichen Implantationsverlauf zu planen. Dies betrifft sowohl den Ablauf der einzelnen Schritte der Implantation als auch den physischen Verlauf, d.h. die relative Lage, der Implantate im Körper. Übliche Bildgebungsverfahren, die hierfür kombiniert werden können, sind beispielsweise normales oder dreidimensionales Röntgen, MRT, CT, optische Intraoralscans, optische Scans von Zahnabdrücken oder in selteneren Fällen auch Ultraschall. Auf diese Art können die Vorteile der verschiedenen Bilgebungsmethoden miteinander kombiniert werden. So ist beispielsweise die Präzision von optischen Methoden, welche die Oberflächengeometrie erfassen, radiologischen Methoden in der Regel weit überlegen. Während radiologische Methoden, wie beispielsweise das 3D-Röntgen, lediglich eine Genauigkeit von mehreren hundert Mikrometern haben, kann mit optischen Intraoralscannern eine Genauigkeit von 10µm oder weniger erreicht werden. Allerdings bieten radiologische Methoden den Vorteil, dass so auch Bereiche erfasst werden können, die nicht optisch erfassbar sind. Dabei lassen sich nicht nur der Verlauf von Knochen und eventuellen Hohlräumen in diesen erfassen, sondern auch Fett, Muskeln und vor allem der Verlauf von Blutgefäßen und Nervensträngen.

Durch das Kombinieren aller Informationen können Implantate geplant werden, die nicht nur perfekt in die sichtbare Zahnreihe passen, sondern auch die Beschädigung umliegender Zahnwurzeln, Nerven und dergleichen vermeiden. Dadurch wird nicht nur die Ästhetik des fertigen Implantates erhöht, sondern auch die Sicherheit der Behandlung und der Komfort für den Patienten. Insbesondere Schäden an Blutgefäßen und Nerven können längerfristige Komplikationen verursachen und den Heilungsprozess erheblich verlängern.

Ein wesentlicher Teil einer fertig geplanten Implantation ist der Verlauf, also die Tiefe und Orientierung der Bohrung, in der das Implantat später verankert wird, im Verhältnis zu Zähnen, Wurzeln, (Kiefer-)Knochen oder auch künstlichem oder natürlichem Knochenaufbau. Hierfür werden im Stand der Technik üblicherweise Schablonen erstellt, die dem Bohrer den Verlauf vorgeben. Diese Bohrschablonen haben jedoch mehrere Nachteile. Werden sie lediglich auf den Zähnen und intraoralen Weichteilen abgelegt, können sie verrutschen und sind unpräzise. Werden sie auf den Zähnen fest verankert, ist hierfür zusätzliche Zeit notwendig, welche die Behandlung für den Patienten unangenehmer gestaltet. Zusätzlich sind auch die Zeit zum Fertigen der Schablone und die Material- bzw. Fertigungskosten zu berücksichtigen. Ein weiterer wesentlicher Nachteil bei der Verwendung dieser Bohrschablonen ist, dass diese den Bereich, in welchem gebohrt wird, in der Regel nahezu vollständig abdecken und so eine effektive Kühlung beim Bohren verhindern. Dadurch kann es sowohl zu Schädigungen des natürlichen oder künstlichen Materials um die Bohrung herum als auch am Bohrer selbst kommen.

Aufgabe der Erfindung ist es daher, die oben beschriebenen Nachteile zu überwinden.

Gelöst wird diese Aufgabe durch ein Verfahren mit den Merkmalen des Anspruchs 1.

Dabei wird ein Ist-Zustand von intraoralen Strukturen mit einem Soll-Zustand verglichen. Die intraoralen Strukturen können den gesamten Mundraum mit Zähnen, (gegebenenfalls festem) Zahnersatz (Kiefer-)Knochen und Weichteilen, wie Zahnfleisch, Muskeln, Blutgefäßen und Nervenbahnen umfassen.

Der Soll-Zustand kann dabei neben den Informationen über die geplante Implantation und insbesondere den Verlauf der geplanten Bohrung sowie die Oberflächengeometrien der umliegenden Zähne auch gesammelte Daten über Gewebe- und Knochenstruktur beinhalten. Neben den optisch erfassbaren bzw. erfassten Oberflächengeometrien können die gesammelten Daten auch geometrische Informationen zu nicht sichtbaren bzw. nicht zugänglichen Bereichen umfassen. Unter "sichtbar" wird im Sinne der Erfindung "für einen optisch arbeitenden Intraoralscanner sichtbar", d.h. vereinfacht auch "optisch erfassbar", verstanden.

Erfindungsgemäß beinhaltet der Ist-Zustand eine Referenzbohrung. Diese Bohrung kann wesentlich kleiner gestaltet werden als die eigentliche Bohrung, in welche später ein Implantat eingesetzt wird. Dies hat den Vorteil, dass eine kleinere Referenzbohrung im Falle eines Fehlers einfacher wieder geschlossen werden kann und dass diese für den Patienten weniger unangenehm ist und weniger Schaden anrichten kann. Für die Funktionalität des erfindungsgemäßen Verfahrens ist die Größe der Referenzbohrung allerdings unerheblich.

Um den Ist- und den Soll-Zustand zu vergleichen, muss zunächst der Ist-Zustand ermittelt werden.

In einem ersten Schritt zum Ermitteln des Ist-Zustandes werden eine Geometrie eines Referenzobjektes und eine Geometrie des Soll-Zustandes zur Verfügung gestellt. Die Geometrie des Soll-Zustandes muss dabei lediglich die sichtbare Oberflächengeometrie des Soll-Zustandes im Bereich der geplanten Implantation umfassen. Es können aber auch umfassendere Daten, die die gesamte Oberflächengeometrie des Soll-Zustandes beinhalten, oder auch der vollständige Soll-Zustand zur Verfügung gestellt werden. Im Sinne einer schnellen rechnerischen Verarbeitung des Vergleiches ist es aber vorteilhaft, nur die Oberflächengeometrie im Bereich der Implantation zur Verfügung zu stellen. Dieser Bereich kann dabei beispielsweise Teile der Oberflächengeometrie der umgebenden Zähne umfassen.

Als Referenzobjekt eignet sich im Prinzip jedes Objekt, das groß genug ist, um die Referenzbohrung auszufüllen, und klein genug, um in den Mund eines Patienten gebracht zu werden. Wichtig ist jedoch, dass die Oberflächengeometrie des Referenzobjektes genau bekannt ist. Dabei kann es sich beispielsweise um einen einfachen Pin mit geeigneten Dimensionen handeln. Es kann aber auch die Geometrie eines Bohrers oder eines Bohrerkopfes hinterlegt sein und zur Verfügung gestellt werden.

Wie bereits erwähnt, ist es zunächst nur erforderlich, dass von der Geometrie des Soll-Zustandes die Oberflächengeometrien der optisch sichtbaren Elemente, insbesondere der umliegenden Zähne, zur Verfügung gestellt werden. Dies kann beispielsweise vorteilhaft sein, um die Geschwindigkeit, mit der das Verfahren durchgeführt werden kann, zu erhöhen, da so erheblich weniger Daten geladen werden müssen. Grundsätzlich kann aber auch der vollständige, geplante Soll-Zustand geladen werden. Die Oberflächenstruktur umliegender Zähne zu wählen, hat den Vorteil, dass die Position der Zähne relativ zum übrigen Kiefer bzw. Mundraum in der Regel konstant ist bzw. implantologische Maßnahmen in der Regel nur gesetzt werden, wenn es zwischen der Planung der Implantation und der Implantation selbst keine Änderungen an diesen gegeben hat. Demgegenüber können sich beispielsweise weiche Gewebe, beispielsweise durch Schwellungen oder auch das Abschwellen derselben im Zuge eines Heilungsprozesses, verändert haben bzw. innerhalb kurzer Zeit ändern.

In einem nächsten Schritt wird, nachdem die Referenzbohrung hergestellt wurde, das Referenzobjekt in der Referenzbohrung positioniert.

Wurde der Bohrer ggf. auch mit dem Bohrkopf als Referenzobjekt mit seiner Oberflächengeometrie hinterlegt, kann dieser natürlich auch einfach in der Referenzbohrung verbleiben. Auch dies gilt im Sinne der Erfindung als "in der Referenzbohrung positioniert". Ist das Referenzobjekt ein einfacher Pin, wird dieser nach dem Entfernen des Bohrers in der Referenzbohrung positioniert. Im einfachsten Fall wird der Pin lediglich in die Referenzbohrung gesteckt.

Dann werden die Oberflächengeometrien der intraoralen Strukturen in der Umgebung der Implantation, insbesondere von Zähnen und/oder anderen ortsfesten Strukturen, wenigstens bereichsweise erfasst. Zeitgleich wird die Oberflächengeometrie des Referenzobjektes wenigstens bereichsweise erfasst. Vorzugsweise erfolgt das Erfassen durch einen optisch arbeitenden Intraoralscanner. Dadurch, dass die Oberflächengeometrie des Referenzobjektes bekannt ist, kann aus dem gescannten Bereich des Referenzobjektes die Lage des kompletten Referenzobjektes extrapoliert werden. Dasselbe gilt für die nicht sichtbaren Teile der intraoralen Strukturen, deren Lage anhand der ortsfesten Komponenten des gescannten Bereiches der intraoralen Strukturen hergeleitet werden kann. Da die sichtbaren Bereiche von Referenzobjekt und intraoralen Strukturen zeitgleich gescannt wurden, kann anhand der räumlichen Verhältnisse der gescannten Bereiche auch das räumliche Verhältnis des nicht sichtbaren Anteils des Referenzobjektes zu den nicht sichtbaren intraoralen Strukturen ermittelt werden.

Da sich das Referenzobjekt nur dort befinden kann, wo sich keine intraoralen Strukturen (mehr) befinden, kann aus dem zuvor ermittelten räumlichen Verhältnis der Verlauf der Referenzbohrung ermittelt werden. Der auf diese Art ermittelte Verlauf der Referenzbohrung zeigt den Ist-Zustand.

In einem anschließenden Schritt werden dann der Verlauf der Referenzbohrung und der Verlauf der geplanten Bohrung verglichen. Daraus kann dann, beispielsweise von einem Zahnarzt oder einem Kieferchirurgen, geschlossen werden, ob Korrekturen vorgenommen werden müssen.

In einer bevorzugten Weiterbildung der Erfindung können der geplante Verlauf und die Referenzbohrung zeitgleich in einem 3D-Modell des Soll-Zustandes visualisiert werden. So lassen sich eventuelle Abweichungen besonders einfach erfassen.

Bevorzugte Ausführungsformen der Erfindung sind Gegenstand der Unteransprüche.

Weitere Merkmale und Vorteile der Erfindung ergeben sich aus der nachfolgenden Beschreibung bevorzugter, den Schutzbereich nicht beschränkender, Ausführungsbeispiele der Erfindung unter Bezugnahme auf die angeschlossenen Zeichnungen. Es zeigt:
- Fig. 1: symbolisch dargestellt eine Aufnahme einer Zahnreihe,
- Fig. 2: die Aufnahme von Fig. 1 mit einem geplanten Implantat,
- Fig. 3: symbolisch die Zahnreihe von Fig. 1 mit einer Referenzbohrung,
- Fig. 4: die Zahnreihe von Fig. 3 mit einem Referenzobjekt,
- Fig. 5: symbolisch eine Aufnahme der Oberflächengeometrie der Zahnreihe mit dem Referenzobjekt, von Fig. 4 und
- Fig. 6: eine Visualisierung von Ist- und Sollzustand.

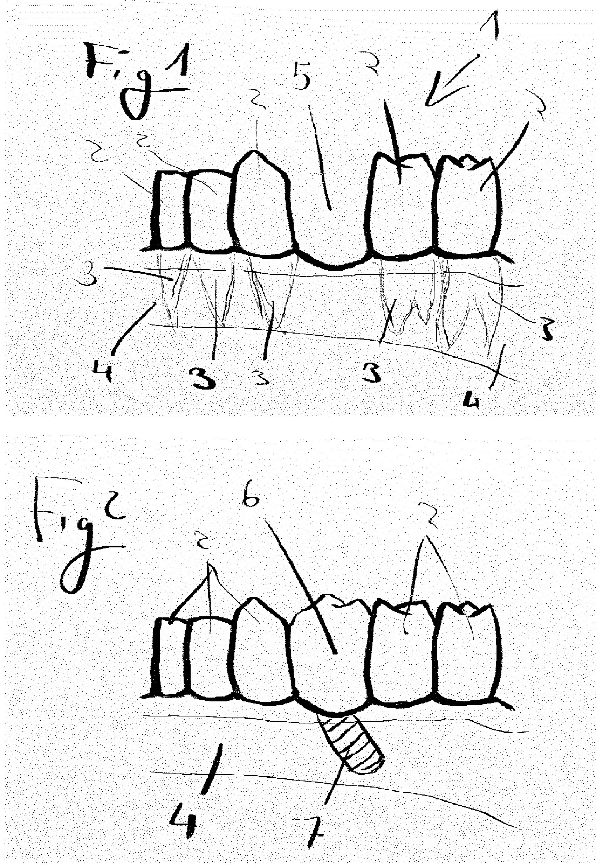

Die in Fig. 1 symbolisch dargestellte Aufnahme einer Zahnreihe 1 zeigt mehrere Zähne 2, die mit ihren Wurzeln 3 in einem Kiefer 4 sitzen. Zwischen den Zähnen 2 ist eine Lücke 5 sichtbar, die durch ein Implantat gefüllt werden soll.

Zahnwurzeln 3 und Kiefer 4 sind dabei in der Aufnahme sichtbar, da Informationen aus verschiedenen Bildgebungsverfahren zusammengeführt wurden.

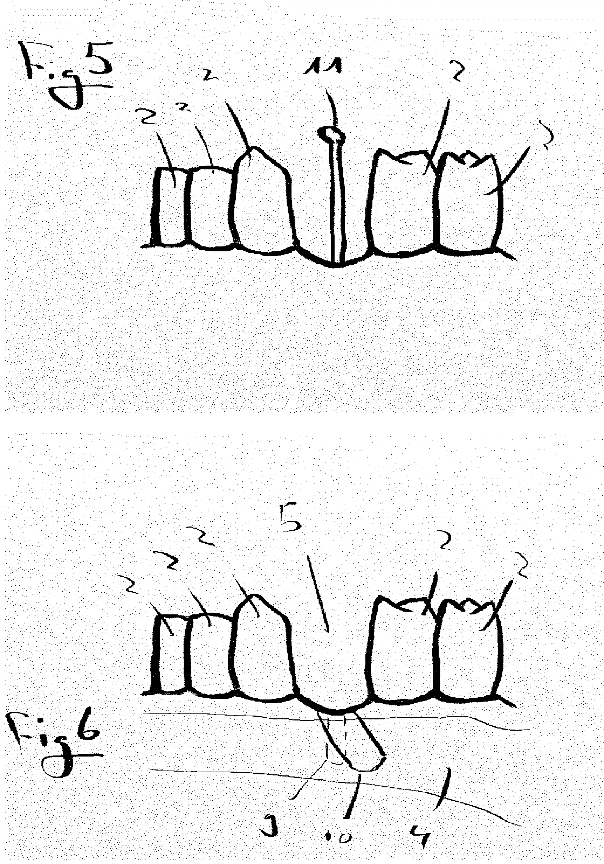

Fig. 2 zeigt vereinfacht die Aufnahme von Fig. 1 mit einem geplanten Implantat 6. Das Implantat 6 ist dabei mit einem Stift 7 im Kiefer 4 verankert. In der in den Figuren gezeigten, beispielhaften Ausführungsform der Erfindung entspricht der Verlauf 10 (siehe Fig. 6) des Stiftes 7 einem Soll-Zustand, der für das Durchführen der Implantation erreicht werden soll. Die Behandlung selbst und auch das Planen des Verlaufs sind nicht Gegenstand der Erfindung, sondern erfolgen, gegebenenfalls technisch unterstützt, gesondert durch entsprechend geschultes Personal aufgrund der zuvor gemessenen, anatomischen Gegebenheiten.

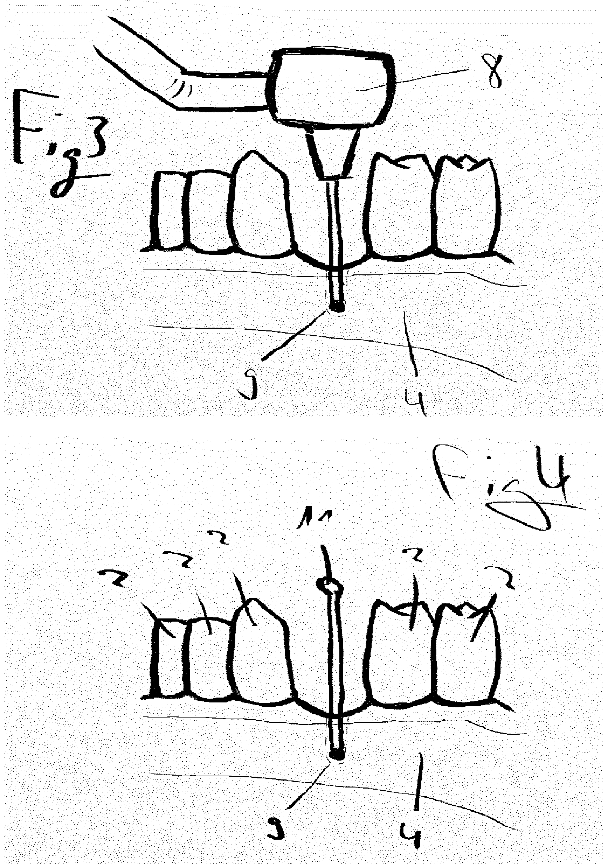

Um den Soll-Zustand mit dem Ist-Zustand zu vergleichen, ist es notwendig, zunächst den Ist-Zustand zu ermitteln. Fig. 3 zeigt symbolisch, wie zunächst mit einem Bohrkopf 8 eine Referenzbohrung 9 (strichliert dargestellt) im Kiefer 4 erzeugt wird.

Verglichen werden sollen in der Folge der reale Verlauf der Referenzbohrung 9 und der geplante Verlauf 10 des Stiftes 7. Dazu wird zunächst, wie in Fig. 4 gezeigt, ein Referenzobjekt 11 in die Referenzbohrung eingebracht. Wichtig ist dabei lediglich, dass die Geometrie des Referenzobjektes 11 bekannt ist. Es kann also auch der in Fig. 3 dargestellte Bohrer als Referenzobjekt dienen.

Dann wird der Bereich mit einem optischen 3D-Scanner erfasst. Dabei kann der Scanner, wie in Fig. 5 illustriert, lediglich die sichtbaren Oberflächengeometrien der Zähne 2 und des Referenzobjektes 11 erfassen. Da die räumliche Anordnung von Zähnen 2 und Kiefer 4 zueinander bekannt ist, kann daraus in Kombination mit der bekannten Geometrie des Referenzobjektes 11 der Verlauf der Referenzbohrung 9 ermittelt werden.

In einem letzten, in Fig. 6 illustrierten Schritt werden dann der ermittelte Verlauf der Referenzbohrung 9 und der geplante Verlauf 10 gemeinsam visualisiert. Aufgrund der Visualisierung kann dann von einer entsprechenden Fachperson entschieden werden, ob eine in der Folge durchzuführende Bohrung, welche dem Verlauf der nicht sichtbaren Referenzbohrung 9 folgt, der Planung entspricht oder nicht.

Zusammenfassend lässt sich die Erfindung wie folgt beschreiben:
Bei einem Verfahren zum Darstellen eines Ist-Zustandes von intraoralen Strukturen und eines Soll-Zustandes, wobei der Soll-Zustand einen Verlauf einer geplanten Bohrung innerhalb einer intraoralen Struktur enthält, wobei die geplante Bohrung optisch erfassbare und optisch nicht erfassbare Bereiche beinhaltet, beinhaltet der Ist-Zustand eine Referenzbohrung. Das Referenzobjekt ist in der Referenzbohrung positioniert und eine Geometrie eines Referenzobjektes und eine Geometrie des Soll-Zustandes werden zur Verfügung gestellt. Dann werden eine Oberflächengeometrie der intraoralen Strukturen und zeitgleich die Oberflächengeometrie des Referenzobjektes wenigstens bereichsweise erfasst. Daraus wird das räumliche Verhältnis der intraoralen Strukturen zum Referenzobjekt ermittelt. Aus dem räumlichen Verhältnis wird der Verlauf der Referenzbohrung ermittelt. Der Verlauf der Referenzbohrung und der Verlauf der geplanten Bohrung werden schließlich in einer Darstellung dargestellt.

## Patentansprüche

1. Verfahren zum Darstellen eines Ist-Zustandes von intraoralen Strukturen und eines Soll-Zustandes, wobei der Soll-Zustand einen Verlauf einer geplanten Bohrung innerhalb einer intraoralen Struktur enthält und wobei die geplante Bohrung optisch erfassbare und optisch nicht erfassbare Bereiche beinhaltet, **dadurch gekennzeichnet, dass** der Ist-Zustand eine Referenzbohrung beinhaltet, dass das Referenzobjekt in der Referenzbohrung positioniert ist, dass eine Geometrie eines Referenzobjektes zur Verfügung gestellt wird, dass eine Geometrie des Soll-Zustandes zur Verfügung gestellt wird, dass dann eine Oberflächengeometrie der intraoralen Strukturen wenigstens bereichsweise erfasst wird, dass zeitgleich die Oberflächengeometrie des Referenzobjektes wenigstens bereichsweise erfasst wird, dass daraus das räumliche Verhältnis der intraoralen Strukturen zum Referenzobjekt ermittelt wird, dass aus dem räumlichen Verhältnis der Verlauf der Referenzbohrung ermittelt wird, und dass der Verlauf der Referenzbohrung und Verlauf der geplanten Bohrung in einer Darstellung dargestellt werden.

2. Verfahren nach Anspruch 1, **dadurch gekennzeichnet, dass** das bereichsweise Erfassen der Oberflächengeometrie der intraoralen Strukturen und der Oberflächengeometrie des Referenzobjektes zeitgleich durch einen optischen Intraoralscanner erfolgt.

3. Verfahren nach Anspruch 1 oder 2, **dadurch gekennzeichnet, dass** Referenzbohrung und geplante Bohrung miteinander vergleichbar, überlappend visualisiert werden.
